(19) Europäisches Patentamt
European Patent Office
Office européen des brevets

(11) EP 2 197 829 B1

(12) FASCICULE DE BREVET EUROPEEN

(45) Date de publication et mention de la délivrance du brevet:
**04.05.2016 Bulletin 2016/18**

(21) Numéro de dépôt: **08840252.4**

(22) Date de dépôt: **24.09.2008**

(51) Int Cl.:
***C07C 209/10*** (2006.01) ***C07C 209/22*** (2006.01)
***C07C 211/46*** (2006.01) ***C07C 211/58*** (2006.01)
***C07D 213/73*** (2006.01) ***B01J 31/22*** (2006.01)

(86) Numéro de dépôt international:
**PCT/FR2008/051701**

(87) Numéro de publication internationale:
**WO 2009/050366 (23.04.2009 Gazette 2009/17)**

(54) **PROCEDE DE SYNTHESE D'ARYLAMINES**

VERFAHREN ZUR SYNTHESE VON ARYLAMIN

ARYLAMINE SYNTHESIS METHOD

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **28.09.2007 FR 0706827**

(43) Date de publication de la demande:
**23.06.2010 Bulletin 2010/25**

(73) Titulaire: **Centre National de la Recherche Scientifique (CNRS)
75016 Paris (FR)**

(72) Inventeurs:
- **TAILLEFER, Marc
F-34570 Vailhauques (FR)**
- **XIA, Ning
F-34296 Montpellier (FR)**

(74) Mandataire: **Colombet, Alain André et al
Cabinet Lavoix
62, rue de Bonnel
69448 Lyon Cedex 03 (FR)**

(56) Documents cités:
**EP-A- 0 549 263**
**WO-A-2007/109365**
**DE-C- 586 879**
**FR-A- 2 238 698**
**US-A- 1 840 760**
**US-A- 2 455 932**
**US-A1- 2001 047 013**

- **SHEN Q ET AL: "Palladium-catalyzed coupling of ammonia and lithium amide with aryl halides" JOURNAL OF THE AMERICAN CHEMICAL SOCIETY, AMERICAN CHEMICAL SOCIETY, WASHINGTON, DC., US, vol. 128, no. 31, 12 juillet 2006 (2006-07-12), pages 10028-10029, XP002459529 ISSN: 0002-7863 cité dans la demande**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne un procédé de préparation d'arylamines et en particulier un procédé de préparation d'aniline et d'anilines substituées sur le noyau aromatique, à partir d'ammoniaque, dans des conditions douces, facilement industrialisables, avec de bonnes sélectivités et de bons rendements.

L'aniline (ou phénylamine) et ses dérivés, qui seront dénommés dans le présent exposé sous le terme générique d'arylamines, sont des composés d'une importance capitale en synthèse organique, notamment comme intermédiaires de synthèse dans de nombreux domaines, dont les colorants, l'industrie pharmaceutique, l'industrie phytosanitaire, et pour la préparation d'isocyanates aromatiques.

L'aniline est aujourd'hui principalement synthétisée par nitration du benzène, puis hydrogénation catalytique du nitrobenzène obtenu. Il s'agit donc d'une synthèse en deux étapes qui pourrait avantageusement être simplifiée, de manière à diminuer les coûts de production, tout en s'affranchissant de certains produits toxiques, comme par exemple les catalyseurs métalliques utilisés dans l'étape d'hydrogénation.

Par ailleurs, l'ammoniac est un des composés chimiques inorganiques le plus produit au monde. Il est principalement utilisé dans la production de fertilisants, d'explosifs, et de composés organiques azotés.

L'ammoniac pourrait donc être avantageusement utilisé pour la préparation de l'aniline et de ses dérivés. Il n'existe cependant à l'heure actuelle que peu d'exemples de réactions de couplage catalytique qui utilisent l'ammoniac comme réactif.

La réaction dite de Ullmann catalysée au cuivre est une des méthodes les plus utilisées dans l'industrie en raison du coût attractif du cuivre, en comparaison avec les coûts d'autres métaux nobles tels que le palladium, le ruthénium, et autres. Cette réaction fait intervenir un composé aromatique porteur d'un groupement partant avec un composé nucléophile porteur d'un hétéroatome (tel que l'atome d'azote) susceptible de se substituer au groupement partant, créant ainsi une liaison carbone-hétéroatome (par exemple carbone-azote).

Des études récentes illustrent les difficultés de l'utilisation d'ammoniac (gazeux ou liquide, c'est-à-dire l'ammoniaque) comme nucléophile azoté, en présence d'un dérivé aromatique et d'un catalyseur, pour la préparation d'aniline et dérivés d'aniline, selon le schéma réactionnel dit de Ullmann.

Par exemple, le document WO 2003/006420, ainsi que la publication de Hartwig et coll. (J. A. C. S., 128, (2006), 10028-9), proposent des réactions d'amination de dérivés aromatiques avec de l'ammoniaque, en présence de complexes à base de palladium en tant que catalyseurs. Ces réactions sont complexes, peu économiques (utilisation de palladium, ligands onéreux), et réalisées sous des pressions élevées. L'extrapolation au plan industriel apparaît donc difficilement envisageable.

De même, les conditions opératoires décrites dans la demande de brevet WO 2004/052833 (températures : 200°C ; pressions : 69 à 90 bars) rendent le procédé peu rentable sur le plan industriel. En outre le catalyseur utilisé est un couple oxyde de cuivre/dioxyde de titane, onéreux, et le milieu réactionnel comprend éventuellement du benzène, qui est un solvant toxique.

Le brevet EP-B-1511726 propose quant à lui l'amination de trichlorobenzène en présence d'iodure de cuivre et d'ammoniaque en milieu aqueux. Cette réaction est toutefois limitée à un dérivé aromatique hyperactivé et est effectuée à hautes températures (180°C) sous de fortes pressions (40 bars). Il est connu d'utiliser du sulfate de cuivre pour la synthèse de composés de type N-alkyl-3,4-dialkyloxyaniline (EP 0 549 263) pour la synthèse d'aminopyridine (DE 586879) et pour la synthèse d'aniline (US 1 840 760). Il est également connu un procédé de préparation d'amines primaires aromatiques comprenant la réaction entre un agent d'arylation comprenant un groupe partant lié à un noyau aromatique, de l'ammoniaque et un complexe comprenant un métal ou un ion du groupe VIII et un ligand dérivé du ferrocène (WO 2007/109365).

Lang et coll. (Tetrahedron Letters, 42, (2001), 3251-3254 & US 2001/0047013) décrivent un procédé d'amination d'halogénures aromatiques par catalyse au cuivre. Toutefois, les précurseurs aromatiques bromés sont nécessairement activés et les sélectivités observées sont faibles.

Ainsi, un premier objectif de la présente invention consiste à proposer un procédé de préparation d'arylamines qui s'affranchisse des inconvénients connus de l'état de la technique.

Un autre objectif de présente invention est de proposer un procédé économique, facilement industrialisable et peu toxique permettant la préparation d'arylamines, dans des conditions douces, c'est-à-dire à des températures et des pressions proches ou relativement proches des conditions normales de température et de pression.

Un autre objectif encore consiste à proposer un procédé de préparation d'arylamines avec des rendements et des sélectivités élevés.

Comme autre objectif, la présente invention vise à proposer un procédé de préparation d'arylamines mettant en oeuvre un catalyseur non ou faiblement toxique, aisé à préparer et de coût relativement faible, suffisamment faible pour être rentable sur le plan industriel.

Comme autre objectif, la présente invention vise à proposer un procédé de préparation d'arylamines de mise en oeuvre aisée, facilement industrialisable, et adaptable à une grande variété de substrats aromatiques, sans modification importante des conditions opératoires, permettant la synthèse d'arylamines de tous types, par exemple non substituées (aniline), ou substituées, ou encore comportant un ou plusieurs cycles aromatiques non substitués (par exemples aminonaphtalènes) ou substitués.

D'autres objectifs encore apparaîtront à la lumière de la description et des exemples qui suivent.

Il a maintenant été découvert que les objectifs définis ci-dessus peuvent être atteints en totalité ou en partie grâce au procédé de la présente invention qui est exposée ci-après.

Ainsi, la présente invention a tout d'abord pour objet un procédé de préparation d'arylamines de formule $R^0$-$(NH_2)_m$, où $R^0$ représente un radical aromatique et m est compris entre 1 et 3 , ledit procédé comprenant les étapes suivantes :

a) préparation d'un milieu réactionnel comprenant :

1) un composé aromatique porteur d'au moins un groupe partant, de formule $R^0$-$Y_m$, dans lequel $R^0$ est un radical aromatique et Y représente un atome d'halogène, m étant compris entre 1 et 3 ;
2) une solution aqueuse d'ammoniaque ;
3) un système catalytique comprenant un complexe métal/ligand choisi parmi les complexes Fe/β-dicétone et/ou Cu/β-dicétone ;
4) une base ; et
5) un solvant organique polaire aprotique;

b) chauffage dudit milieu réactionnel à une température comprise entre 20°C et 200°C;
c) conduite de la réaction ; et
d) extraction et isolement de l'arylamine $R^0$-$(NH_2)_m$ formée.

Dans un mode de réalisation préféré, le métal est le cuivre.

Dans un autre mode de réalisation, le métal est le fer.

Dans un mode de réalisation, la présente invention a pour objet un procédé tel que précédemment défini dans lequel le composé aromatique porteur d'un groupe partant est un composé de formule $R^0$-Y, dans lequel $R^0$ est un radical aromatique et Y représente un atome d'halogène.

Le schéma général du procédé selon la présente invention peut être illustré comme suit lorsque n est égal à 1 :

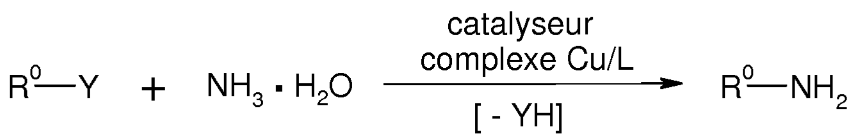


schéma dans lequel $R^0$-Y représente un composé aromatique porteur d'un groupe partant Y.

Selon le procédé de la présente invention, on réalise une réaction d'arylation en faisant réagir un composé aromatique porteur d'au moins un groupe partant avec une solution aqueuse d'ammoniaque, c'est-à-dire du gaz ammoniac dissous dans de l'eau.

Dans l'exposé qui suit de la présente invention, le terme « arylation » est utilisé dans son sens large, puisqu'on envisage la mise en oeuvre d'un composé aromatique porteur d'un groupement partant qui est soit de type aromatique carbocyclique ou soit de type aromatique hétérocyclique.

Plus précisément, dans le composé aromatique porteur d'un groupement partant $R^0$-Y, $R^0$ représente un radical aromatique ou hétéroaromatique contenant de 2 à 20 atomes, monocyclique ou polycyclique.

Y représente un atome d'halogène.

Selon un autre aspect, comme groupes partants préférés, on choisit de préférence un atome d'halogène, de préférence brome, chlore ou iode.

Selon un mode de réalisation préféré de l'invention, le composé aromatique porteur d'un groupement partant $R^0$-Y est représenté par la formule (A) :

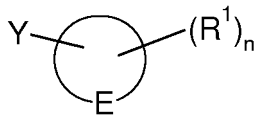

(A)

dans laquelle :

- E symbolise le reste d'un cycle formant tout ou partie d'un système carbocyclique et/ou hétérocyclique, aromatique, monocyclique ou polycyclique ;
- $R^1$, identiques ou différents, représentent des substituants sur le cycle ;
- Y représente un atome d'halogène tel que précédemment défini ; et
- n représente le nombre de substituants sur le cycle.

L'invention s'applique notamment aux composés halogénoaromatiques répondant à la formule (A) dans laquelle E est le reste d'un composé cyclique, ayant de préférence au moins 5 atomes dans le cycle, de préférence 5 ou 6, éventuellement substitué, et représentant au moins l'un des cycles suivants :

- un carbocycle aromatique, monocyclique ou polycyclique, c'est-à-dire un composé constitué par au moins 2 carbocycles aromatiques et formant entre eux des systèmes *ortho-* ou *ortho-* et péri-condensés ou un composé constitué par au moins 2 carbocycles dont l'un seul d'entre eux est aromatique et formant entre eux des systèmes *ortho-* ou *ortho*-et péri-condensés ;

- un hétérocycle aromatique, monocyclique comportant au moins un des hétéroatomes P, O, N et/ou S, ou un hétérocycle aromatique polycyclique, c'est-à-dire un composé constitué par au moins 2 hétérocycles contenant au moins un hétéroatome dans chaque cycle dont au moins l'un des deux cycles est aromatique et formant entre eux des systèmes *ortho-* ou *ortho-* et *péri*-condensés ; ou un composé constitué par au moins un carbocycle et au moins un hétérocycle dont au moins l'un des cycles est aromatique et formant entre eux des systèmes *ortho-* ou *ortho-* et *péri*-condensés ;

Plus particulièrement, le reste E éventuellement substitué représente préférentiellement le reste d'un carbocycle aromatique, tel que le benzène, d'un bicycle aromatique comprenant deux carbocycles aromatiques, tel que le naphtalène, ou un bicycle partiellement aromatique comprenant deux carbocycles dont l'un deux est aromatique, tel que le tétrahydro-1,2,3,4-naphtalène.

L'invention envisage également le fait que E peut représenter le reste d'un hétérocycle. Comme exemples particuliers, on peut citer un hétérocycle aromatique tel que le furane, la pyridine ; un bicycle aromatique comprenant un carbocycle aromatique et un hétérocycle aromatique, tel que le benzofurane, la benzopyridine ; un bicycle partiellement aromatique comprenant un carbocycle aromatique et un hétérocycle, tel que le méthylènedioxybenzène ; un bicycle aromatique comprenant deux hétérocycles aromatiques, tel que la 1,8-naphtylpyridine ; un bicycle partiellement aromatique comprenant un carbocycle et un hétérocycle aromatique, tel que la tétrahydro-5,6,7,8-quinoléine.

Dans le procédé de l'invention, on met en oeuvre préférentiellement un composé halogénoaromatique de formule (A) dans laquelle E représente un noyau aromatique, de préférence un noyau benzénique ou naphtalénique.

Le composé aromatique de formule (A) peut être porteur d'un ou plusieurs substituants. Dans le présent texte, on entend par « plusieurs », généralement, moins de 4 substituants, c'est-à-dire que, dans la formule (A), n représente 0, 1, 2, 3, 4 ou 5, généralement n représente 0, 1, 2, 3 ou 4, de préférence n représente 0 ou 1, voire 2.

Le ou les éventuel(s) substituant(s) $R^1$ du reste E est(sont) de tout type connu en soi et en particulier choisi(s) parmi la liste des substituants suivante, sans que cette liste ne présente de caractère limitatif

- un groupe alkyle, linéaire ou ramifié, de $C_1$ à $C_6$, de préférence de $C_1$ à $C_4$ atomes de carbone, tel que méthyle, éthyle, propyle, isopropyle, butyle, isobutyle, sec-butyle, tert-butyle ;
- un groupe alcényle ou alcynyle, linéaire ou ramifié, de $C_2$ à $C_6$, de préférence, de $C_2$ à $C_4$, tel que vinyle, allyle ;
- un groupe alkoxy ou thioéther linéaire ou ramifié, de $C_1$ à $C_6$, de préférence de $C_1$ à $C_4$ tel que les groupes méthoxy, éthoxy, propoxy, isopropoxy, butoxy, un groupe alcényloxy, de préférence, un groupe allyloxy ou un groupe phénoxy ;
- un groupe cyclohexyle, phényle ou benzyle ;
- un groupe ou une fonction tel que hydroxyle, thiol, carboxyle, ester, amide, formyle, acyle, aroyle, amide, urée, isocyanate, thioisocyanate, nitrile, azoture, nitro, sulfone, sulfonique, halogène, pseudohalogène ou trifluorométhyle.

$R^1$ peut également représenter un radical hétérocyclyle saturé, insaturé ou aromatique comprenant 5 ou 6 atomes et comprenant soufre, oxygène et/ou azote, en tant qu'hétéroatome(s). À cet égard, on peut citer en particulier les groupes pirydyle, pyrazolyle ou imidazolyle.

Comme exemples de composés répondant à la formule (A), et plus généralement à la formule $R^0$-Y, on peut citer notamment le fluorobenzène, le chlorobenzène, l'iodobenzène, le bromobenzène, le *para*-chlorotoluène, le *para*-bromotoluène, le *para*-bromoanisole, le *méta*-bromoanisole, le *para*-iodo-anisole, le para-cyano-bromobenzène, le *para*-cyano-iodobenzène, le *para*-bromotrifluorobenzène, le *para*-bromophényl-benzène, le 1-iodonaphtalène, le *pa-*

*ra*-bromo(méthylcarbonyl)benzène, la 3-bromopyridine, le para-iodo-nitrobenzène, l'ortho-iodobenzoate de méthyle, le 1-bromo-naphtalène, le *para*-bromométhyl-benzène et la 2-bromopyridine.

Comme exemple de composés répondant à la formule A et plus généralement à la formule $R^0$-$(Y)_2$, comprenant deux groupes partants, on peut citer notamment, l'*ortho*-difluorobenzène, le *meta*-difluorobenzène, le para-difluorobenzène, l'*ortho*-dichlorobenzène, le *meta*-dichlorobenzène, le para-dichlorobenzène, l'*ortho*-diiodobenzène, le *meta*-diiodobenzène, le para-diiodobenzène, l'*ortho*-dibromobenzène, le *meta*-dibromobenzène, le para-dibromobenzène, l'*ortho*-chlorofluorobenzène, *l'ortho*-iodofluorobenzène, l'*ortho*-bromofluorobenzène, le *meta*-chlorofluorobenzène, le *meta*-iodofluorobenzène, le *meta*-bromofluorobenzène, le *meta*-chlorofluorobenzène, le *meta*-iodofluorobenzène, le *meta*-bromobenzène, le para-chlorofluorobenzène, le *para*-iodofluorobenzène, le para-bromofluorobenzène, *l'ortho*-iodochlorobenzène, l'*ortho*-bromochlorobenzène, le *meta*-iodochlorobenzène, le *meta*-bromochlorobenzène, le *para*-iodochlorobenzène, le para-bromochlorobenzène, *l'ortho*-bromoiodobenzène, le *meta*-bromoiodobenzène et le *para*-bromoiodobenzène.

Les composés aromatiques porteurs d'un groupement partant de formule $R^0$-$Y_m$ définis ci-dessus sont soit disponibles dans le commerce, soit aisément accessibles à partir de produits connus et de modes opératoires connus ou décrits dans la littérature scientifique, la littérature brevets, les Chemical Abstracts ou l'Internet.

Selon le procédé de l'invention, le composé aromatique porteur d'un groupe partant est mis en contact avec de l'ammoniaque, c'est-à-dire une solution d'ammoniac dans l'eau, préparée selon les techniques connues de l'homme du métier, ou disponible directement dans le commerce. Les solutions aqueuses ammoniacales utilisables dans le cadre de l'invention sont des solutions de concentrations variables, et en général comprises entre 1% à 30% en poids, avantageusement supérieures à 25%, de préférence une solution d'ammoniaque à environ 28%.

Conformément au procédé de l'invention, on fait réagir de l'ammoniaque avec un composé aromatique porteur d'un groupe partant, en présence d'un système catalytique comprenant un complexe cuivre/ligand.

Il a en effet été découvert qu'il est possible de réaliser des réactions d'amination de composés aromatiques, telles que définies *supra,* entre l'ammoniaque et des composés aromatiques porteurs d'un groupe partant, en utilisant un système catalytique comprenant un complexe métal/ligand.

On entend par complexe cuivre/ligand un complexe constitué par du cuivre 0, I, II ou III et un ligand organique, à l'exclusion des sels de cuivre.

Dans un mode de réalisation, le complexe cuivre/ligand est constitué par du cuivre I ou II et un ligand organique, à l'exclusion des sels de cuivre.

On entend par complexe fer/ligand un complexe constitué par du fer 0, I, II ou III et un ligand organique, à l'exclusion des sels de fer.

Dans un mode de réalisation, le complexe fer/ligand est constitué par du fer II ou III et un ligand organique, à l'exclusion des sels de fer

Comme exemples de systèmes catalytiques susceptibles d'être mis en oeuvre, on peut citer ceux comprenant au moins un complexe cuivre/ligand ou fer/ligand, c'est-à-dire des complexes du cuivre avec au moins un ligand ou du fer avec au moins un ligand.

Les complexes cuivre/ligand ou fer/ligand utilisables dans la présente invention sont bien connus de l'homme du métier. Ils sont soit disponibles dans le commerce, soit facilement préparés à partir de composés connus et disponibles selon des modes opératoires connus dans la littérature scientifique, la littérature brevets, les Chemical Abstracts ou l'Internet.

Par exemple, les complexes à base de cuivre définis plus haut peuvent être préparés par mise en contact d'au moins un ligand, avec le cuivre métallique ou un dérivé du cuivre (cuivre(0), cuivre (1),cuivre(II) ou cuivre (III), par exemple un halogénure de cuivre, tel que l'iodure, le bromure ou le chlorure cuivrique ou cuivreux, ou d'autres dérivés.

Par exemple, les complexes à base de fer définis plus haut peuvent être préparés par mise en contact d'au moins un ligand, avec le fer métallique ou un dérivé du fer (fer (0), fer (1), fer(II) ou fer(III)), par exemple un halogénure de fer, tel que l'iodure, le bromure ou le chlorure ferrique ou ferreux, ou d'autres dérivés.

La formation du complexe est généralement effectuée sous atmosphère inerte, par exemple sous azote ou argon, en milieu solvant organique, de préférence un solvant polaire aprotique, par exemple l'acétonitrile ou le DMF. Cette réaction de complexation est habituellement conduite à une température comprise entre 0°C et 80°C, selon la nature des composés en présence, et généralement la température de réaction est la température ambiante.

Le complexe est généralement obtenu sous la forme d'un précipité qui est isolé du milieu réactionnel selon des techniques connues en soi, par exemple par filtration, et éventuellement recristallisation dans un solvant, avantageusement identique à celui utilisé pour la réaction de complexation.

Selon une variante, le complexe métal/ligand, cuivre/ligand ou fer/ligand peut être préparé *in situ,* dans le milieu réactionnel de la réaction de préparation d'arylamines selon l'invention.

Les complexes métal/ligand sont des complexes du cuivre (0), du cuivre (I), du cuivre (II), du cuivre (III), du fer (0), du fer (I), du fer (II) ou du fer (III). Le ligand est choisi dans le groupe constitué par les β-dicétones. Parmi les ligands de type β-dicétone, on peut citer ceux choisis dans le groupe constitué par la pentane-2,4-dione (acétylacétonate), la

1,5-diphénylpentane-2,4-dione, la 3-méthylpentane-2,4-dione, la 1-(N,N-diméthyl)aminobutane-1,3-dione, la 2-acétylcyclohexanone, la 2,2,6,6-tétraméthylheptane-3,5-dione, et la 1-éthoxybutane-1,3-dione, sans que cette liste ne constitue une quelconque limitation.

Dans un mode de réalisation, le ligand est l'acétylacétonate (acac) formant, avec le cuivre, le complexe cuivre (II) / acétylacétonate, ou $Cu(acac)_2$.

Il peut également être envisagé d'utiliser le complexe cuivre/ligand tel que défini ci-dessus en association avec un ou plusieurs autres composés du cuivre, notamment ceux choisis parmi le cuivre métallique, les oxydes de cuivre (I) ou de cuivre (II), les hydroxydes de cuivre (I) ou de cuivre (II), les sels organiques ou inorganiques de cuivre (I) ou de cuivre (II).

À titre d'exemples non limitatifs, de tels composés du cuivre peuvent être choisis parmi le cuivre (0), les halogénures de cuivre (par exemple l'iodure de cuivre (I), le bromure de cuivre (I), le bromure de cuivre (II), le chlorure de cuivre (I), le chlorure de cuivre (II)), les oxydes ou hydroxydes de cuivre (par exemple l'oxyde de cuivre (1), l'oxyde de cuivre (II), l'hydroxyde de cuivre (II)), les nitrates de cuivre (par exemple le nitrate de cuivre (I), le nitrate de cuivre (II)), les sulfates ou sulfites de cuivre (par exemple le sulfate de cuivre (I), le sulfate de cuivre (II), le sulfite de cuivre (I)), les sels organiques de cuivre, dans lesquels le contre-ion comprend au moins un atome de carbone (par exemple le carbonate de cuivre (II), l'acétate de cuivre (I), l'acétate de cuivre (II), le trifluorométhylsulfonate de cuivre (II), le méthylate de cuivre (I), le méthylate de cuivre (II).

Ainsi, un système catalytique convenable pour le procédé de la présente invention peut être avantageusement l'acétylacétonate de cuivre (II), seul ou en association avec un ou plusieurs autres catalyseurs à base de cuivre, et notamment ceux choisis parmi le cuivre métallique (0) (Cu), l'iodure de cuivre (I) (CuI), et l'oxyde de cuivre (II) (CuO).

Dans le présent texte, on se réfère ci-dessus et dans la suite à la Classification périodique des éléments publiée dans le Bulletin de la Société Chimique de France, n°1 (1966).

Ainsi, un intérêt du procédé de l'invention est de faire appel à une catalyse par le cuivre plutôt que le palladium ou le nickel, c'est-à-dire un catalyseur moins toxique et apportant en outre un avantage du point de vue économique.

Selon une variante, l'invention n'exclut pas que le cuivre soit associé à une faible quantité d'un autre élément métallique désigné par M. L'élément métallique M est choisi dans le groupe (VIII), (IB) et (IIB) de la classification périodique des éléments, telle que définie plus haut.

Comme exemples de métaux M, on peut citer l'argent, le palladium, le cobalt, le nickel, le fer et/ou le zinc, le manganèse.

On utilise avantageusement un mélange comprenant du palladium et du cuivre. Le palladium peut être apporté sous forme d'un métal finement divisé ou sous forme d'un dérivé inorganique tel qu'un oxyde ou un hydroxyde. Il est possible de faire appel à un sel minéral de préférence, nitrate, sulfate, oxysulfate, halogénure, oxyhalogénure, silicate, carbonate, ou à un dérivé organique de préférence, cyanure, oxalate, acétylacétonate ; alcoolate et encore plus préférentiellement méthylate ou éthylate ; carboxylate et encore plus préférentiellement acétate.

Peuvent être également mis en oeuvre des complexes, notamment chlorés ou cyanés de palladium et/ou de métaux alcalins, de préférence sodium, potassium ou d'ammonium. À titre d'exemples de composés susceptibles d'être mis en oeuvre pour la préparation des catalyseurs de l'invention, on peut citer notamment le bromure de palladium(II), le chlorure de palladium(II), l'iodure de palladium(II), le cyanure de palladium(II), le nitrate de palladium (II) hydraté, l'oxyde de palladium(II), le sulfate de palladium(II)dihydraté, l'acétate de palladium(II), le propionate de palladium(II), le butyrate de palladium(II), le benzoate de palladium.

Comme exemples spécifiques de dérivés du nickel, on peut citer les halogénures de nickel(II), tels que chlorure, bromure ou iodure de nickel(II) ; le sulfate de nickel(II) ; le carbonate de nickel(II) ; les sels d'acides organiques comprenant de 1 à 18 atomes de carbone tels que notamment acétate, propionate ; les complexes de nickel(II) tels que l'acétylacétonate de nickel(II), le dibromo-bis-(triphénylphosphine) de nickel (II), le dibromo-bis (bipyridine) de nickel(II) ; les complexes de nickel(0) tels que le bis-(cycloocta-1,5-diène) de nickel(0), le bis-diphénylphosphinoéthane de nickel(0).

On peut également faire appel à des dérivés à base de fer ou de zinc, généralement sous la forme d'oxyde, d'hydroxydes ou de sels tels que les halogénures, de préférence le chlorure, les nitrates et les sulfates.

La quantité de l'élément métallique M représente moins de 50 %, de préférence moins de 25, avantageusement moins de 10 % molaire par rapport au nombre de moles de cuivre.

Encore plus préférentiellement, on met en oeuvre dans le procédé de l'invention un système catalytique comprenant uniquement du cuivre, en tant que métal, sous forme de complexe avec un ligand. Un complexe tout particulièrement préféré est l'acétylacétonate de cuivre ($Cu(acac_2)$).

Le ou les complexe(s) présent(s) dans le système catalytique mis en oeuvre dans le procédé de l'invention peu(ven)t éventuellement être supporté(s), comme cela est connu dans le domaine, par exemple sur support minéral, silice, alumine ou autres supports, notamment à base d'oxydes métalliques ou non métalliques.

La quantité totale de catalyseur complexe cuivre/ligand mise en oeuvre dans le procédé de l'invention, exprimée par le ratio molaire entre le nombre de moles de complexe exprimé en cuivre, et le nombre de moles de composé aromatique porteur d'un groupe partant, varie généralement entre 0,001 et 1, de préférence entre 0,01 et 0,1.

Intervient également, dans le procédé de l'invention, une base dont la fonction est de piéger le groupe partant.

Les bases convenables pour le procédé de l'invention peuvent être caractérisées par leur pKa, qui est avantageusement au moins supérieur ou égal à 2, de préférence compris entre 4 et 30.

Le pKa est défini comme la constante de dissociation ionique du couple acide/base, lorsque l'eau est utilisée comme solvant. Pour le choix d'une base ayant un pKa tel que défini par l'invention, on peut se reporter, entre autres, au Handbook of Chemistry and Physics, 66e édition, pp. D-161 et D-162.

Parmi les bases utilisables, on peut citer entre autres, les bases minérales telles que les carbonates, hydrogénocarbonates, phosphates ou hydroxydes de métaux alcalins, de préférence de sodium, de potassium, de césium, ou de métaux alcalino-terreux, de préférence de calcium, baryum ou magnésium.

On peut également faire appel aux hydrures de métaux alcalins, de préférence l'hydrure de sodium ou aux alcoolates de métaux alcalins, de préférence de sodium ou de potassium, et plus préférentiellement au méthylate, éthylate ou *tert*-butylate de sodium.

Conviennent également des bases organiques comme les amines tertiaires et l'on peut citer plus particulièrement la triéthylamine, la tri-n-propylamine, la tri-n-butylamine, la méthyldibutylamine, la méthyl-dicyclohexylamine, l'éthyldi-isopropylamine, la N,N-diéthylcyclohexylamine, la pyridine, la diméthylamino-4pyridine, la N-méthylpipéridine, la N-éthylpipéridine, la N-n-butylpipéridine, la 1,2-diméthylpipéridine, la N-méthylpyrrolidine, et la 1,2-diméthylpyrrolidine.

Parmi les bases, on choisit préférentiellement les carbonates de métaux alcalins, avantageusement le carbonate de sodium, le carbonate de potassium, et tout particulièrement le carbonate de césium.

La quantité de base mise en oeuvre est telle que le ratio entre le nombre de moles de base et le nombre de moles du composé aromatique porteur du groupe partant varie préférentiellement entre 0,5 et 4, de préférence égal à environ 2.

La réaction de couplage, notamment la réaction d'amination d'un composé aromatique selon l'invention, est conduite en présence d'un solvant organique. On fait de préférence appel à un solvant organique, qui ne réagit pas dans les conditions de la réaction.

Comme types de solvants mis en oeuvre dans le procédé de l'invention, on fait appel à un solvant organique polaire aprotique.

Des exemples, non limitatifs, de solvants qui peuvent être mis en oeuvre dans le procédé de l'invention, sont choisis parmi :

- les carboxamides linéaires ou cycliques comme le N,N-diméthyl-acétamide (DMAC), le N,N-diéthylacétamide, le diméthylformamide (DMF), le diéthylformamide ou la 1-méthyl-2-pyrrolidinone(NMP) ;
- le diméthylsulfoxyde (DMSO) ;
- l'hexaméthylphosphotriamide (HMPT) ;
- la tétraméthylurée ;
- les composés nitrés tels que le nitrométhane, le nitroéthane, le 1-nitropropane, le 2-nitropropane ou leurs mélanges, le nitrobenzène ;
- les nitriles aliphatiques ou aromatiques comme l'acétonitrile, le propionitrile, le butanenitrile, l'iso-butanenitrile, le pentanenitrile, le 2-méthylglutaronitrile, l'adiponitrile ;
- la tétraméthylène sulfone (sulfolane) ;
- les carbonates organiques tels que le diméthylcarbonate, le di-isopropylcarbonate, le di-n-butylcarbonate ;
- les esters d'alkyle tels que l'acétate d'éthyle ou d'isopropyle ;
- les hydrocarbures aromatiques halogénés ou non, tels que le chlorobenzène ou le toluène ;
- les cétones telles que l'acétone, la méthyléthylcétone, la méthylisobutylcétone, la cyclopentanone, la cyclohexanone ;
- les hétérocycles azotés tels que la pyridine, la picoline et les quinoléines.

On peut également utiliser un mélange de deux ou plusieurs solvants, notamment choisis parmi ceux listés ci-dessus.

Les solvants préférés sont les carboxamides, tels que le DMF, l'acétonitrile, le DMSO, la NMP et le DMAC, de manière tout à fait préférée, le DMF et/ou l'acétonitrile.

La quantité de solvant organique à mettre en oeuvre est déterminée en fonction de la nature du solvant organique choisi. Elle est déterminée de telle sorte que la concentration du composé porteur du groupe partant dans le solvant organique soit de préférence comprise entre 1% et 40% en poids.

Selon un autre aspect, la quantité du ou des solvant(s) introduit(s) dans le milieu réactionnel est telle que le ratio pondéral quantité de solvant/quantité totale d'eau (y compris l'eau de la solution aqueuse d'ammoniac) est généralement compris entre 1 et 50, de préférence entre 3 et 30.

Comme indiqué précédemment, la réaction selon l'invention met en oeuvre une solution aqueuse d'ammoniaque. Le solvant de la réaction est avantageusement soluble dans l'eau, notamment l'eau de la solution aqueuse d'ammoniaque. En variante, le solvant peut être ajouté au milieu réactionnel en mélange avec une quantité additionnelle d'eau.

Selon une autre variante, le composé nucléophile et/ou le composé porteur du groupe partant peu(ven)t être utilisé(s) comme solvant(s) de la réaction, auquel cas il n'est pas nécessaire d'ajouter un solvant supplémentaire au milieu réactionnel.

La quantité du composé aromatique porteur d'un groupe partant mise en oeuvre est généralement exprimée par rapport à la quantité d'ammoniac (nombre de moles de $NH_3$) et peut varier dans de grandes proportions ; généralement elle est voisine de la stoechiométrie.

Ainsi, le rapport entre le nombre de moles du composé aromatique porteur du groupe partant et le nombre de moles d'ammoniac (nombre de moles de $NH_3$) varie le plus souvent entre 0,01 et 2,0, de préférence entre 0,05 et 1,0, et encore plus préférentiellement entre 0,1 et 0,3.

Un autre avantage du procédé de l'invention est d'effectuer la réaction à température modérée.

La réaction d'amination du composé aromatique selon le procédé de l'invention est généralement conduite à une température qui est avantageusement située entre 20°C et 200°C, de préférence entre 30°C et 130°C, et encore plus préférentiellement entre 40°C et 110°C.

Ladite réaction est généralement mise en oeuvre sous pression atmosphérique, ou sous légère surpression, mais des pressions plus élevées pouvant atteindre par exemple 10 bars peuvent être également utilisées. De très bons rendements et de très bonnes sélectivités ont été obtenus à des pressions inférieures à 1.38 bars.

En outre la réaction selon l'invention peut être conduite sans nécessité d'atmosphère inerte. Toutefois, ladite réaction peut être effectuée sous azote, argon ou autre gaz inerte communément utilisé en synthèse organique.

D'un point de vue pratique, la réaction est simple à mettre en oeuvre.

Un autre avantage est de pouvoir utiliser une large gamme de composés aromatiques porteurs d'un groupe partant, non seulement les iodures, mais aussi les bromures ou les chlorures notamment les iodures d'aryle, les bromures d'aryle ou les chlorures d'aryle

L'ordre de mise en oeuvre des réactifs n'est pas critique. De préférence, on charge le système catalytique complexe cuivre/ligand, l'ammoniaque, le composé aromatique porteur du groupe partant, et éventuellement la base et le solvant organique. Le milieu réactionnel est alors porté à la température souhaitée.

Comme mentionné précédemment, on peut, en variante, introduire le cuivre, et au moins un ligand, afin de former le complexe cuivre/ligand *in situ.*

On contrôle l'avancement de la réaction en suivant la disparition du composé aromatique porteur du groupe partant. En fin de réaction, on obtient un produit du type $R^0$-$NH_2$, $R^0$ étant tel que défini précédemment.

La durée de la réaction varie selon plusieurs paramètres, dont les quantités et natures des réactifs, catalyseurs, et solvants mis en oeuvre. En outre, la durée de la réaction dépend de la température à laquelle elle est effectuée. En règle générale, la durée de réaction peut varier de quelques minutes à plusieurs heures, voire quelques dizaines d'heures, plus généralement entre 1 heure et 20 heures.

Le composé obtenu est finalement récupéré selon les techniques classiques utilisées, notamment par cristallisation dans un solvant organique, lorsque le produit obtenu est un solide.

Comme exemples plus spécifiques de tels solvants organiques utilisables dans l'étape de cristallisation, on peut mentionner notamment les hydrocarbures aliphatiques ou aromatiques, halogénés ou non, les carboxamides et les nitriles. On peut citer notamment le cyclohexane, le toluène, le diméthylformamide, l'acétonitrile.

Comme autre avantage, le procédé selon l'invention permet ainsi d'obtenir des arylamines $R^0$-$NH_2$, à partir de précurseurs aromatiques porteurs d'un groupe partant $R^0$-Y, où $R^0$ et Y sont tels que définis précédemment, de manière aisée, en particulier en une seule étape (« one pot reaction »).

En particulier, le procédé de l'invention permet d'obtenir sélectivement l'aniline en une seule étape, à partir d'un halogénure de phényle et d'ammoniaque, avec de bons rendements, à une température de 90°C à pression atmosphérique, en présence d'un système catalytique comprenant un complexe Cu/ligand, et d'une base, en milieu solvant eau/DMF.

L'invention est maintenant illustrée au moyen des exemples qui suivent et qui ne présentent aucun caractère limitatif.

## Exemples

### Exemple A : Synthèse de 1-aminonaphtalène

Dans un tube de schlenk ou un tube de Radley (préalablement purgé à l'azote et rempli d'azote), on introduit le complexe cuivre/acétylacétonate ($[Cu(acac)_2]$ ; 52 mg ; 0,2 mmol), de l'acétylacétonate (82 μL ; 0,8 mmol), du 1-iodonaphtalène (508 mg ; 2 mmol) et du carbonate de césium ($Cs_2CO_3$ ; 978 mg ; 3,0 mmol).

Du diméthylformamide (DMF ; 4 mL) est alors ajouté sous azote, puis de l'ammoniaque à 28% (en poids) (600 μL). Le tube est scellé sous azote et le mélange est chauffé à 90°C et mis sous agitation pendant 24 heures. Après

refroidissement jusqu'à température ambiante, le mélange est dilué au dichlorométhane, puis lavé à l'eau. La phase aqueuse est extraite cinq fois au dichlorométhane. Les phases organiques sont combinées, séchées sur sulfate de sodium ($Na_2SO_4$), puis concentrées pour fournir le produit brut.

Ce produit brut est purifié par chromatographie sur gel de silice (éluant cyclohexane/acétate d'éthyle, 9:1) pour fournir le 1-aminonaphtalène, sous la forme d'un solide.

**<u>Exemple B</u> : Synthèse de 4-Aminobenzonitrile**

Dans un tube de schlenk ou un tube de Radley (préalablement purgé à l'azote et rempli d'azote), on introduit le cuivre acétate ($Cu(OAc)_2$ ; 36 mg ; 0,2 mmol), du 3-Methyl-2,4-pentanedione (156 µL ; 1,2 mmol), du 4-iodobenzonitrile (458 mg ; 2 mmol) et du carbonate de césium ($Cs_2CO_3$ ; 978 mg ; 3,0 mmol).

Du diméthylformamide (DMF ; 4 mL) est alors ajouté sous azote, puis de l'ammoniaque à 28% (en poids) (600 µL). Le tube est scellé sous azote et le mélange est chauffé à 90°C et mis sous agitation pendant 24 heures. Après refroidissement jusqu'à température ambiante, le mélange est dilué au dichlorométhane, puis lavé à l'eau. La phase aqueuse est extraite cinq fois au dichlorométhane. Les phases organiques sont combinées, séchées sur sulfate de sodium ($Na_2SO_4$), puis concentrées pour fournir le produit brut.

Ce produit brut est purifié par chromatographie sur gel de silice (éluant cyclohexane/acétate d'éthyle, 9:1) pour fournir le 4-aminobenzonitrile, sous la forme d'un solide.

**<u>Exemple C :</u> Synthèse de 3-Anisidine**

Dans un tube de schlenk ou un tube de Radley (préalablement purgé à l'azote et rempli d'azote), on introduit le complexe cuivre/acétylacétonate ($[Cu(acac)_2]$ ; 52 mg ; 0,2 mmol), de l'acétylacétonate (82 µL ; 0,8 mmol), du 3-bromoanisole (374 mg ; 2 mmol) et du carbonate de césium ($Cs_2CO_3$ ; 978 mg ; 3,0 mmol).

Du diméthylformamide (DMF ; 4 mL) est alors ajouté sous azote, puis de l'ammoniaque à 28% (en poids) (600 µL). Le tube est scellé sous azote et le mélange est chauffé à 90°C et mis sous agitation pendant 24 heures. Après refroidissement jusqu'à température ambiante, le mélange est dilué au dichlorométhane, puis lavé à l'eau. La phase aqueuse est extraite cinq fois au dichlorométhane. Les phases organiques sont combinées, séchées sur sulfate de sodium ($Na_2SO_4$), puis concentrées pour fournir le produit brut.

Ce produit brut est purifié par chromatographie sur gel de silice (éluant cyclohexane/acétate d'éthyle, 9:1) pour fournir la 3-anisidine, sous la forme d'une huile.

**<u>Exemple D :</u> Influence du composé porteur du groupement partant**

Pour tester l'étendue de la réaction d'amination, divers essais ont été effectués selon les mêmes modes opératoires que ceux décrits ci-dessus, à partir de différents halogénures d'aryle, porteurs de substituants soit électro-attracteurs soit électro-donneurs.

Les résultats sont présentés dans le Tableau 3.

**<u>-- Tableau 3 - Réactions avec divers précurseurs aromatiques --</u>**

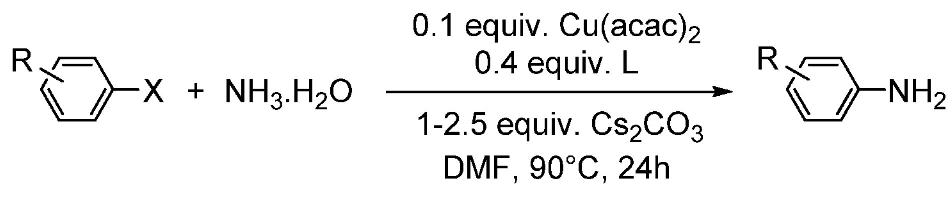


| *N°* | *ArX* | *ArNH₂* | *Rendement‡ (%)* |
|---|---|---|---|
| D1 | 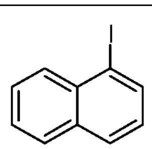 | 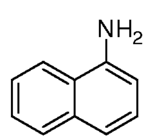 | 90 |
| D2 | 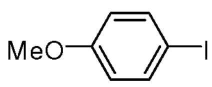 | 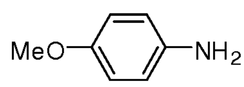 | 80 |

(suite)

| *N°* | *ArX* | *ArNH$_2$* | *Rendement‡ (%)* |
|---|---|---|---|
| D3 | 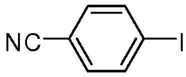 | 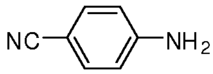 | 93**, 99†,97* |
| D4 | 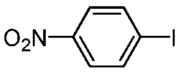 | 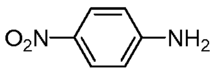 | 63 |
| D5 | 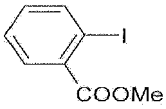 | 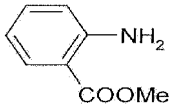 | 23 |
| D6 | PhBr | $PhNH_2$ | 78 |
| D7 | 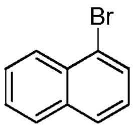 | 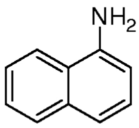 | 88 |
| D8 | 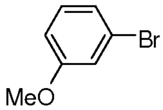 | 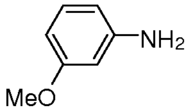 | 85 |
| D9 | 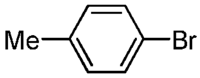 | 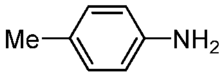 | 65, 79*** |
| D10 | 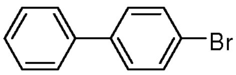 | 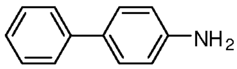 | 90, 98†† |
| D11 | 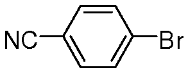 | 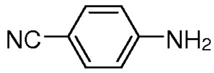 | 92, (82 *and**) |
| D12 | 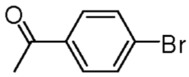 | 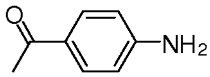 | 92, 84** |
| D13 | 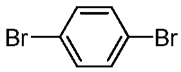 | 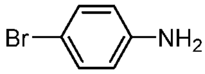 | 41 ††† |
| D14 | 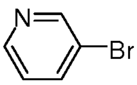 | 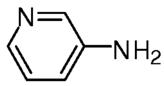 | 84 |
| D15 | 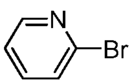 | 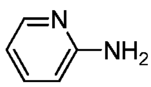 | 82 |

Les rendements sont les rendements isolés. Sauf indication contraire la 2,4-pentanedione est utilisée comme ligand. † avec la 3-méthyl-2,4-pentanedione ou la 2,2,6,6-tétraméthyl-3,5-heptanedione utilisées comme ligand. * Réaction réalisée à 70°C. ** Réaction réalisée à 60°C *** Réaction temps 36 h. †† Réalisée avec la tétraméthyl-3,5-heptanedione ††† Formation du produit de double substitution la *para*-aminoaniline 42%.

**<u>Exemple E :</u> Influence du ligand**

Pour tester l'influence de la nature des ligands sur la réaction d'amination, divers essais ont été effectués à partir de 4-bromobiphényle en présence de complexes de cuivre avec différents ligands.

Les résultats sont présentés dans le Tableau 4.

**-- Tableau 4 - Réactions en présence de complexes de cuivre avec différents ligands --**

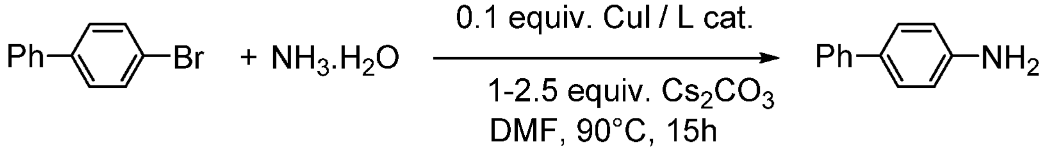


| N° | ***Ligands L*** | ***Rendement‡ (%)*** | ***Selectivité†*** |
|---|---|---|---|
| E1 | 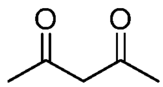 | 76 | 92 |
| E2 | 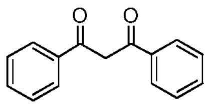 | 24 | 92 |
| E3 | 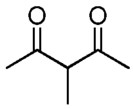 | 7 | - |
| E4 | 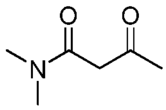 | 29 | 91 |
| E5 | 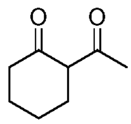 | 35 | 99 |
| E6 | 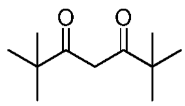 | 79 | 99 |
| E7 | 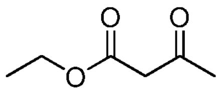 | 7 | - |
| E8 | 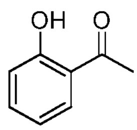 | 0 | - |

***‡* L** (0.6 eq) et $NH_3$ aqueux commercial à 28% (5 eq) ont été utilisés. Rendement déterminé en utilisant le 1,3-diméthoxybenzène comme standard. † Selectivité / couplage C-C entre L et le 4-bromobiphényle.

**Exemple F : Influence de la source du cuivre et des solvants**

Pour tester l'influence de la source de cuivre et des solvants, divers essais ont été effectués à partir du 4-bromobiphényl de complexe Cu(acac)$_2$ en présence de divers sels de cuivre et de divers solvants.

-- Tableau 5 - Réactions en présence de divers sels de cuivre et solvants

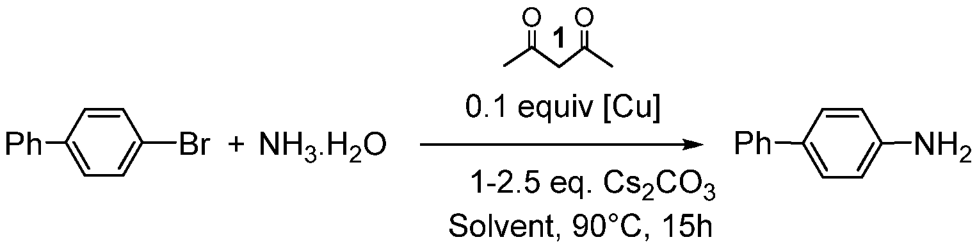


| | [Cu], 0.1 eq. | Ligand **1** (eq.) | solvant | Rendement [a] [%] |
|---|---|---|---|---|
| F1 | - | 0.6 | DMF | 0 |
| F2 | CuI | - | DMF | 0[b] |
| F3 | CuI | 0.6 | DMF | 2[c] |
| F4 | CuI | 0.6 | DMF | 76 |
| F5 | Cu | 0.6 | DMF | 68 |
| F6 | CuO | 0.6 | DMF | 79 |
| F7 | $Cu(OAc)_2$ | 0.6 | DMF | 73 |
| F8 | $Cu_2O$ | 0.6 | DMF | 63 |
| F9 | $Cu(acac)_2$ | - | DMF | 23 |
| F10 | $Cu(acac)_2$ | 0.4 | DMF | 76 |
| F11 | $Cu(acac)_2$ | 0.4 | DMSO | 18 |
| F12 | $Cu(acac)_2$ | 0.4 | $CH_3CN$ | 34 |
| F13 | $Cu(acac)_2$ | 0.4 | NMP | 50 |
| F14 | $Cu(acac)_2$ | 0.4 | $H_2O$ | 0 |
| F15 | $Cu(acac)_2$ | 0.4 | DMF | 6[d] |
| F16 | $Cu(acac)_2$ | 0.4 | DMF | 45[e] |
| F17 | $Cu(acac)_2$ | 0.4 | DMF | 45[f] |
| F18 | $Cu(acac)_2$ | 0.4 | DMF | 93[g] , 20[h] |

[a] Rendement déterminé en utilisant le 1,3-diméthoxy benzène comme standard interne. [b] Même resultat à 140°C. [c] Sans base. [d] DMF présaturé avec de l'ammoniac gazeux. [e] Addition de 0.5 eq. de $NBu_4^+Br^-$. [f] $K_2CO_3$ a été utilisé au lieu de $Cs_2CO_3$. [g] Temps de réaction 24 h. [h] Temps de réaction 24 h, 3% de $Cu(acac)_2$ ont été utilisés.

**<u>Exemple G :</u> Synthèse de Aniline catalysée au fer**

Dans un tube de schlenk ou un tube de Radley (préalablement purgé à l'azote et rempli d'azote), on introduit le complexe fer/acétylacétonate ($[Fe(acac)_3]$ ; 70 mg ; 0,2 mmol), du 2,2,6,6-tetraméthyle-3,5-heptanedione (166 µL ; 0,8 mmol), du Iodobenzène (204 mg ; 1 mmol) et du carbonate de césium ($Cs_2CO_3$ ; 489 mg ; 1,5 mmol).

Du diméthylformamide (DMF ; 2 mL) est alors ajouté sous azote, puis de l'ammoniaque à 28% (en poids) (300 µL). Le tube est scellé sous azote et le mélange est chauffé à 140°C et mis sous agitation pendant 24 heures. Après refroidissement jusqu'à température ambiante, le mélange est dilué au dichlorométhane, puis lavé à l'eau. La phase aqueuse est extraite cinq fois au dichlorométhane. Les phases organiques sont combinées, séchées sur sulfate de sodium ($Na_2SO_4$), puis concentrées pour fournir le produit brut.

Ce produit brut est purifié par chromatographie sur gel de silice (éluant cyclohexane/acétate d'éthyle, 9:1) pour fournir l'aniline (20% - 30%), sous la forme d'une huile.

La présente invention fournit ainsi un procédé général, de mise en oeuvre aisée, économique et efficace pour convertir des halogénures d'aryle en dérivés d'aniline, ledit procédé ne comportant qu'une seule étape. De bons rendements, et de bonnes sélectivités, alliés à une grande variété de substituants possibles, rendent ce procédé tout à fait

rentable et susceptible d'application industrielle, notamment dans le domaine de la synthèse organique.

Les coûts relativement faibles à la fois de l'ammoniaque et du cuivre catalytique rendent le procédé selon la présente invention facilement adaptable et rentable sur le plan économique pour une production d'arylamines (aniline et dérivés) industrielle à grande échelle, en particulier là où les questions de sécurité et d'environnement jouent un rôle prépondérant.

## Revendications

**1.** Procédé de préparation d'arylamines de formule $R^0$-$(NH_2)_m$, où $R^0$ représente un radical aromatique et m est compris entre 1 et 3 , ledit procédé comprenant les étapes suivantes :

a) préparation d'un milieu réactionnel comprenant :

1) un composé aromatique porteur d'au moins un groupe partant, de formule $R^0$-$Y_m$, dans lequel $R^0$ est un radical aromatique et Y représente un atome d'halogène, m étant compris entre 1 et 3 ;
2) une solution aqueuse d'ammoniaque ;
3) un système catalytique comprenant un complexe métal/ligand choisi parmi les complexes Fe/β-dicétone et/ou Cu/β-dicétone;
4) une base ; et
5) un solvant organique polaire aprotique ;

b) chauffage dudit milieu réactionnel à une température comprise entre 20°C et 200°C ;
c) conduite de la réaction ; et
d) extraction et isolement de l'arylamine $R^0$-$(NH_2)_m$ formée.

**2.** Procédé selon la revendication 1, dans lequel $R^0$ représente un radical aromatique ou hétéroaromatique, monocyclique ou polycyclique, contenant de 2 à 20 atomes.

**3.** Procédé selon l'une quelconque des revendications précédentes, dans lequel le composé aromatique porteur d'un groupement partant $R^0$-Y est représenté par la formule (A) :

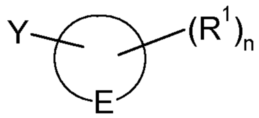

(A)

dans laquelle :

- E symbolise le reste d'un cycle formant tout ou partie d'un système carbocyclique et/ou hétérocyclique, aromatique, monocyclique ou polycyclique ;
- $R^1$, identiques ou différents, représentent des substituants sur le cycle ;
- Y représente un groupe partant tel que défini à la revendication 1; et
- n représente le nombre de substituants sur le cycle.

**4.** Procédé selon la revendication 43, dans laquelle E représente le reste d'un carbocycle aromatique ou d'un hétérocycle aromatique choisi parmi le benzène, le naphtalène, le tétrahydro-1,2,3,4-naphtalène, le furane, la pyridine, le benzofurane, la benzopyridine, le méthylènedioxybenzène, la 1,8-naphtylpyridine et la tétrahydro-5,6,7,8-quinoléine.

**5.** Procédé selon l'une quelconque des revendications précédentes, dans lequel le composé aromatique porteur d'un groupement partant est choisi parmi le fluorobenzène, le chlorobenzène, l'iodobenzène, le bromobenzène, le *para*-chlorotoluène, le *para*-bromotoluène, le *para*-bromoanisole, le *méta*-bromoanisole, le *para*-iodo-anisole, le *para-cyano*-bromobenzène, le *para*-cyano-iodobenzène, le *para*-bromotrifluorobenzène, le *para*-bromophénylbenzène, le 1-iodonaphtalène, le *para*-bromo(méthylcarbonyl)benzène, la 3-bromopyridine, le para-iodo-nitrobenzène, l'*ortho*-iodobenzoate de méthyle, le 1-bromo-naphtalène, le *para*-bromométhyl-benzène et la 2-bromopyridine.

**6.** Procédé selon l'une quelconque des revendications précédentes, dans lequel le composé aromatique porteur de

deux groupes partants est choisi parmi l'*ortho*-difluorobenzène, le *meta*-difluorobenzène, le *para*-difluorobenzène, l'*ortho*-dichlorobenzène, le *meta*-dichlorobenzène, le *para*-dichlorobenzène, l'*ortho*-diiodobenzène, le *meta*-diiodobenzène, le *para*-diiodobenzène, l'*ortho*-dibromobenzène, le *meta*-dibromobenzène, le *para*-dibromobenzène, l'*ortho*-chlorofluorobenzène, l'*ortho*-iodofluorobenzène, l'*ortho*-bromofluorobenzène, le *meta*-chlorofluorobenzène, le *meta*-iodofluorobenzène, le *meta*-bromofluorobenzène, le *meta*-chlorofluorobenzène, le *meta*-iodofluorobenzène, le *meta*-bromobenzène, le *para*-chlorofluorobenzène, le *para*-iodofluorobenzène, le *para*-bromofluorobenzène, l'*ortho*-iodochlorobenzène, l'*ortho*-bromochlorobenzène, le *meta*-iodochlorobenzène, le *meta*-bromochlorobenzène, le *para*-iodochlorobenzène, le *para*-bromochlorobenzène, l'*ortho*-bromoiodobenzène, le *meta*-bromoiodobenzène et le *para*-bromoiodobenzène.

**7.** Procédé selon l'une quelconque des revendications précédentes, dans lequel le complexe cuivre/ligand du système catalytique est un complexe du cuivre (0), (I), (II) ou (III) ou du fer (0), (I), (II) ou (III) avec un ligand de type β-dicétone choisi parmi la pentane-2,4-dione (acétylacétonate), la 1,5-diphénylpentane-2,4-dione, la 3-méthylpentane-2,4-dione, la 1-(N,N-diméthyl)aminobutane-1,3-dione, la 2-acétylcyclohexanone, la 2,2,6,6-tétraméthylheptane-3,5-dione et la 1-éthoxybutane-1,3-dione.

**8.** Procédé selon l'une quelconque des revendications précédentes, dans lequel le système catalytique est le complexe acétylacétonate de cuivre ($Cu(acac_2)$).

**9.** Procédé selon l'une quelconque des revendications précédentes, dans lequel le ratio molaire entre le nombre de moles de complexe exprimé en cuivre, et le nombre de moles de composé aromatique porteur d'un groupe partant, varie entre 0,001 et 1.

**10.** Procédé selon l'une quelconque des revendications précédentes, dans lequel la base présente dans le milieu réactionnel possède un pKa au moins supérieur ou égal à 2.

**11.** Procédé selon l'une quelconque des revendications précédentes, dans lequel la base est choisie parmi les carbonates, hydrogénocarbonates, phosphates ou hydroxydes de métaux alcalins, ou de métaux alcalino-terreux.

**12.** Procédé selon la revendication 12, dans lequel les métaux alcalins sont choisis parmi le sodium, le potassium et le césium et les métaux alcalino-terreux sont choisis parmi le calcium, le baryum et le magnésium.

**13.** Procédé selon l'une quelconque des revendications précédentes, dans lequel la quantité de base mise en oeuvre est telle que le ratio entre le nombre de moles de base et le nombre de moles du composé aromatique porteur du groupe partant est compris entre 0.5 et 4.

**14.** Procédé selon l'une quelconque des revendications précédentes, dans lequel la réaction est effectuée en présence d'un solvant organique polaire aprotique choisi parmi :

- les carboxamides linéaires ou cycliques;
- le diméthylsulfoxyde (DMSO) ;
- l'hexaméthylphosphotriamide (HMPT) ;
- la tétraméthylurée ;
- les composés nitrés;
- les nitriles aliphatiques ou aromatiques ;
- la tétraméthylène sulfone (sulfolane) ;
- les carbonates organiques;
- les esters d'alkyle;
- les hydrocarbures aromatiques halogénés ou non;
- les cétones;
- les hétérocycles azotés.

**15.** Procédé selon l'une quelconque des revendications précédentes, dans lequel le rapport entre le nombre de moles du composé aromatique porteur du groupe partant et le nombre de moles d'ammoniac (nombre de moles de $NH_3$) est compris entre 0,01 et 2,0.

**16.** Procédé selon l'une quelconque des revendications précédentes, dans lequel la réaction est effectuée à une température comprise entre 20°C et 200°C.

**17.** Procédé selon l'une quelconque des revendications précédentes, dans lequel la réaction est conduite sous pression atmosphérique.

**18.** Procédé selon l'une quelconque des revendications précédentes, de préparation de l'aniline en une seule étape, à partir d'un halogénure de phényle et d'ammoniaque, à une température de 90°C, en présence d'un système catalytique comprenant un complexe Cu/β-dicétone, et d'une base, en milieu solvant eau/DMF.

**Patentansprüche**

**1.** Verfahren zur Bereitstellung von Arylaminen der Formel $R^0$-$(NH_2)_m$, wobei $R^0$ ein aromatisches Radikal darstellt und m zwischen 1 und 3 liegt, wobei das Verfahren die folgenden Schritte umfasst:

a) Bereitstellen eines reaktiven Mediums umfassend:

1) eine aromatische Verbindung als Träger mindestens einer Abgangsgruppe der Formel $R^0$-$Y_m$, in der $R^0$ ein aromatisches Radikal und Y ein Halogenatom ist und m zwischen 1 und 3 liegt;
2) eine wässrige Ammoniaklösung;
3) ein katalytisches System, das einen Metall/Ligand-Komplex, gewählt unter den Komplexen Fe/β-Diketon und/oder Cu/β-Diketon, umfasst;
4) eine Base; und
5) ein polares aprotisches organisches Lösungsmittel;

b) Heizen des reaktiven Mediums auf eine Temperatur zwischen 20°C und 200°C;
c) Durchführen der Reaktion; und
d) Extrahieren und Isolieren des gebildeten $R^0$-$(NH_2)_m$ Arylamins.

**2.** Verfahren nach Anspruch 1, bei dem $R^0$ ein aromatisches oder heteroaromatisches, monozyklisches oder polyzyklisches Radikal darstellt, das 2 bis 20 Atome enthält.

**3.** Verfahren nach einem beliebigen der vorhergehenden Ansprüche, bei dem die aromatische Verbindung als Träger einer Abgangsgruppe $R^0$-Y durch die Formel (A) dargestellt ist:

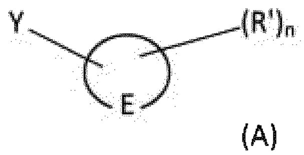

(A)

bei der:

- E den Rest eines Zyklus symbolisiert, der vollständig oder teilweise aus einem carbozyklischen und/oder heterozyklischen, aromatischen, monozyklischen oder polyzyklischen System gebildet ist,
- $R^1$, identisch oder unterschiedlich, die Substituenten an dem Zyklus darstellen;
- Y eine Abgangsgruppe, wie in Anspruch 1 definiert, darstellt; und
- n die Zahl der Substituenten an dem Zyklus, darstellt.

**4.** Verfahren nach Anspruch 3, bei dem E den Rest eines aromatischen Carbozyklus oder eines aromatischen Heterozyklus darstellt, der ausgewählt ist aus Benzol, Naphtalin, Tetrahydro-1, 2, 3, 4-Naphtalin, Furan, Pyridin, Benzofuran, Benzopyridin, Methylendioxybenzol, 1, 8-Naphtylpyridin und Tetrahydro-5, 6, 7, 8-chinolin.

**5.** Verfahren nach einem beliebigen der vorhergehenden Ansprüche, bei dem die aromatische Verbindung als Träger einer Abgangsgruppierung ausgewählt ist aus Fluorbenzol, Chlorbenzol, Iodbenzol, Brombenzol, para-Chlortoluol, para-Bromtoluol, para-Bromanisol, meta-Bromanisol, para-Iodanisol, para-Cyano-Brombenzol, para-Cyano-Iodbenzol, para-Brom-Trifluorbenzol, para-Brom-Phenylbenzol, 1-Iodnaphtalin, para-Brom-(Methylcarbonyl)benzol, 3-Brompyridin, para-Iod-Nitrobenzol, ortho-Iodbenzoat des Methyls, 1-Bromnaphtalin, para-Brom-Methylbenzol und 2-Brompyridin.

6. Verfahren nach einem beliebigen der vorhergehenden Ansprüche, bei dem die aromatische Verbindung als Träger von zwei Abgangsgruppen ausgewählt ist aus ortho-Difluorbenzol, meta-Difluorbenzol, para-Difluorbenzol, ortho-Dichlorbenzol, meta-Dichlorbenzol, para-Dichlorbenzol, ortho-Diiodbenzol, meta-Diiodbenzol, para-Diiodbenzol, ortho-Dibrombenzol, meta-Dibrombenzol, para-Dibrombenzol, ortho-Chlorfluorbenzol, ortho-lodfluorbenzol, ortho-Bromfluorbenzol, meta-chlorfluorbenzol, meta-lodfluorbenzol, meta-Bromfluorbenzol, meta-Chlorfluorbenzol, meta-lodfluorbenzol, meta-Brombenzol, para-Chlorfluorbenzol, para-lodfluorbenzol, para-Bromfluorbenzol, ortholodchlorbenzol, ortho-Bromchlorbenzol, meta-lodchlorbenzol, meta-Bromchlorbenzol, para-lodchlorbenzol, para-Bromchlorbenzol, ortho-Bromiodbenzol, meta-Bromiodbenzol und para-Bromiodbenzol.

7. Verfahren nach einem der vorhergehenden Ansprüche, bei dem der Kupfer/Ligand Komplex des katalytischen Systems ein komplex von Kupfer (0), (I), (II) oder (III) oder Eisen (0), (I), (II) oder (III) mit einem Liganden des Typs β-Diketon, ausgewählt aus Pentan-2, 4-dion (Acetylacetonat), 1,5-Diphenylpentan-2, 4-dion, 3-Methylpentan-2, 4-dion, 1-(N,N-Dimethyl)aminobutan-1, 3-dion, 2-Acetylcyclohexanon, 2, 2,6,6-Tetramethylheptan-3, 5-dion und 1-Ethoxybutan-1, 3-dion, ist.

8. Verfahren nach einem beliebigen der vorhergehenden Ansprüche, bei dem das katalytische System der Acetylacetonat-Komplex ($Cu(acac_2)$) des Kupfers ist.

9. Verfahren nach einem beliebigen der vorhergehenden Ansprüche, bei dem das Molverhältnis zwischen der Molzahl des Komplexes, ausgedrückt in Kupfer, und der Molzahl der aromatischen Verbindung als Träger einer Abgangsgruppe, zwischen 0,001 und 1 variiert.

10. Verfahren nach einem beliebigen der vorhergehenden Ansprüche, bei dem die in dem Reaktionsmedium vorhandene Base einen pKa mindestens größer oder gleich 2 besitzt.

11. Verfahren nach einem beliebigen der vorhergehenden Ansprüche, bei dem die Base ausgewählt ist aus Carbonaten, Hydrogencarbonaten, Phosphaten oder Hydroxiden von Alkalimetallen oder Erdalkalimetallen.

12. Verfahren nach Anspruch 12, bei dem die Alkalimetalle ausgewählt sind aus Natrium, Kalium und Cäsium und die Erdalkalimetalle ausgewählt sind aus Calzium, Barium und Magnesium.

13. Verfahren nach einem beliebigen der vorhergehenden Ansprüche, bei dem die verwendete Basenmenge derart ist, dass das Verhältnis zwischen der Molzahl der Base und der Molzahl der aromatischen Verbindung als Träger der Abgangsgruppe zwischen 0,5 und 4 liegt.

14. Verfahren nach einem beliebigen der vorhergehenden Ansprüche, bei dem die Reaktion durchgeführt wird in Gegenwart eines polaren aprotischen organischen Lösungsmittels ausgewählt aus:

- linearen oder zyklischen Carboxamiden;
- Dimethylsulfoxid (DMSO);
- Hexamethylphosphotriamid (HMPT);
- Tetramethylharnstoff;
- Nitroverbindungen;
- aliphatische oder aromatische Nitrile;
- Tetramethylensulfon (Sulfolan);
- organische Carbonate;
- Alkylester;
- halogenierte oder nichthalogenierte aromatische Kohlenwasserstoffe;
- Ketone;
- stickstoffhaltige Heterozyklen.

15. Verfahren nach einem der vorhergehenden Ansprüche, bei dem das Verhältnis zwischen der Molzahl der aromatischen Verbindung als Träger der Abgangsgruppe und der Molzahl von Ammoniak (Molzahl von $NH_3$) zwischen 0,01 und 2,0 liegt.

16. Verfahren nach einem beliebigen der vorhergehenden Ansprüche, bei dem die Reaktion bei einer Temperatur zwischen 20°C und 200°C durchgeführt wird.

**17.** Verfahren nach einem beliebigen der vorhergehenden Ansprüche, bei dem die Reaktion unter atmosphärischem Druck durchgeführt wird.

**18.** Verfahren nach einem beliebigen der vorhergehenden Ansprüche zur Bereitstellung von Anilin in einem einzigen Schritt aus einem Phenylhalogenid und Ammoniaklösung bei einer Temperatur von 90°C in Gegenwart eines katalytischen Systems umfassend einen Komplex aus Cu/β-Diketon und eine Base in einem Lösungsmittel aus Wasser/DMF.

## Claims

**1.** A process for the preparation of arylamines of formula $R^0$-$(NH_2)_m$, where $R^0$ is an aromatic radical and m is between 1 and 3, said process comprising the following steps:

a) preparation of a reaction medium comprising:

1) an aromatic compound carrying at least one leaving group, of formula $R^0$-$Y_m$, in which $R^0$ is an aromatic radical and Y is a halogen atom, m being between 1 and 3;
2) an aqueous ammonia solution;
3) a catalytic system comprising a metal/ligand complex chosen among Fe/β-dicetone complex and/or Cu/β-dicetone complex;
4) a base; and
5) a polar aprotic organic solvent;

b) heating said reaction medium to a temperature of between 20°C and 200°C;
c) carrying out the reaction; and
d) extracting and isolating the arylamine $R^0$-$(NH_2)_m$ formed.

**2.** The process as claimed in claim 1, in which the $R^0$ radical is an aromatic or heteroaromatic monocyclic or polycyclic radical comprising from 2 to 20 atoms.

**3.** The process as claimed in any one of the preceding claims, in which the aromatic compound carrying a leaving group $R^0$-Y has the formula (A):

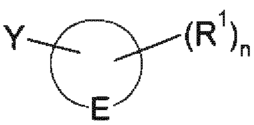

(A)

in which:

- E symbolizes the residue of a ring forming all or part of a carbocyclic and/or heterocyclic system which is aromatic, monocyclic or polycyclic;
- $R^1$, which are identical or different, are substituents on the ring;
- Y is a leaving group as defined in claim 1; and
- n is the number of substituents on the ring.

**4.** The process as claimed in claim 3, in which E is the residue of an aromatic carbocycle or of an aromatic heterocycle selected from benzene, naphthalene, 1,2,3,4-tetrahydronaphthalene, furan, pyridine, benzofuran, benzopyridine, methylenedioxybenzene, 1,8-naphthylpyridine and 5,6,7,8-tetrahydroquinoline.

**5.** The process as claimed in any one of the preceding claims, in which the aromatic compound carrying a leaving group is selected from fluorobenzene, chlorobenzene, iodobenzene, bromobenzene, *para*-chlorotoluene, *para*-bromotoluene, *para*-bromoanisole, *meta*-bromoanisole, *para*-iodoanisole, *para*-cyanobromobenzene, *para*-cyanoiodobenzene, *para*-bromotrifluorobenzene, *para*-bromo-phenylbenzene, 1-iodonaphthalene, *para*-bromo-(methylcarbonyl)benzene, 3-bromopyridine, *para*-iodonitrobenzene, methyl *ortho*-iodobenzoate, 1-bromonaphthalene, *para*-bromomethylbenzene and 2-bromopyridine.

6. The process as claimed in any one of the preceding claims, in which the aromatic compound carrying two leaving groups is selected from ortho-difluorobenzene, meta-difluorobenzene, para-difluorobenzene, ortho-dichlorobenzene, meta-dichlorobenzene, para-dichlorobenzene, ortho-diiodobenzene, meta-diiodobenzene, para-diiodobenzene, ortho-dibromobenzene, meta-dibromobenzene, para-dibromobenzene, ortho-chlorofluorobenzene, ortho-iodofluorobenzene, ortho-bromomethylbenzene, meta-chlorofluorobenzene, meta-iodofluorobenzene, meta-bromofluorobenzene, meta-chlorofluorobenzene, meta-iodofluorobenzene, meta-bromobenzene, para-chlorofluorobenzene, para-iodofluorobenzene, para-bromofluoro-benzene, ortho-iodochlorobenzene, ortho-bromochlorobenzene, meta-iodochlorobenzene, meta-bromochlorobenzene, para-iodochlorobenzene, para-bromochlorobenzene, ortho-bromoiodobenzene, meta-bromoiodobenzene and para-bromoiodobenzene.

7. The process as claimed in any one of the preceding claims, in which the copper/ligand complex of the catalytic system is a complex of copper (0), (I), (II), or (III) or of iron (0), (I), (II) or (III) with a ligand of β-diketone type selected from pentane-2,4-dione(acetylacetone), 1,5-diphenylpentane-2,4-dione, 3-methylpentane-2,4-dione, 1-(N,N-dimethylamino)butane-1,3-dione, 2-acetylcyclohexanone, 2,2,6,6-tetramethylheptane-3,5-dione and 1-ethoxybutane-1,3-dione.

8. The process as claimed in any one of the preceding claims, in which the catalytic system is the copper acetylacetonate ($Cu(acac)_2$) complex.

9. The process as claimed in any one of the preceding claims, in which the molar ratio of the number of moles of complex, expressed as copper, to the number of moles of aromatic compound carrying a leaving group varies between 0.001 and 1.

10. The process as claimed in any one of the preceding claims, in which the base present in the reaction medium has a pKa at least greater than or equal to 2.

11. The process as claimed in any one of the preceding claims, in which the base is selected from carbonates, hydrogenocarbonates, phosphates or hydroxides of alkali metals or of alkaline earth metals.

12. The process according to claim 12, wherein the alkali metal is chosen among sodium, potassium or cesium and the alkaline earth metal is chosen among calcium, barium, or magnesium.

13. The process as claimed in any one of the preceding claims, in which the amount of base employed is such that the ratio of the number of moles of base to the number of moles of the aromatic compound carrying the leaving group is between 0.5 and 4.

14. The process as claimed in any one of the preceding claims, in which the reaction is carried out in the presence of a polar aprotic organic solvent selected from:

- linear or cyclic carboxamides;
- dimethyl sulfoxide (DMSO);
- hexamethylphosphortriamide (HMPT);
- tetramethylurea;
- nitro compounds;
- aliphatic or aromatic nitriles;
- tetramethylene sulfone (sulfolane);
- organic carbonates;
- alkyl esters;
- halogenated or nonhalogenated aromatic hydrocarbons;
- nitrogenous heterocycles.

15. The process as claimed in any one of the preceding claims, in which the ratio of the number of moles of the aromatic compound carrying the leaving group to the number of moles of ammonia (number of moles of $NH_3$) is between 0.01 and 2.0.

16. The process as claimed in any one of the preceding claims, in which the reaction is carried out at a temperature of between 20°C and 200°C.

**17.** The process as claimed in any one of the preceding claims, in which the reaction is carried out at atmospheric pressure.

**18.** The process as claimed in any one of the preceding claims, for the preparation of aniline in a single step, starting from a phenyl halide and from aqueous ammonia, at a temperature of 90°C, in the presence of a catalytic system comprising a Cu/β-dicétone complex and of a base, in a water/DMF solvent medium.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2003006420 A
- WO 2004052833 A
- EP 1511726 B
- EP 0549263 A
- DE 586879
- US 1840760 A
- WO 2007109365 A
- US 20010047013 A


### Littérature non-brevet citée dans la description


- **HARTWIG.** *J. A. C. S.,* 2006, vol. 128, 10028-9
- **LANG.** *Tetrahedron Letters,* 2001, vol. 42, 3251-3254
- *Bulletin de la Société Chimique de France,* 1966
- Handbook of Chemistry and Physics. D-161, , D-162